(19) Europäisches Patentamt
European Patent Office
Office européen des brevets

(11) **EP 3 855 452 A1**

(12) **EUROPEAN PATENT APPLICATION**

(43) Date of publication:
**28.07.2021 Bulletin 2021/30**

(51) Int Cl.:
***G16H 50/20*** (2018.01)　***G06T 7/00*** (2017.01)

(21) Application number: **20153515.0**

(22) Date of filing: **24.01.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **WEN, Dong
5656 AE Eindhoven (NL)**
• **LI, Zuofeng
5656 AE Eindhoven (NL)**
• **TAO, Liang
5656 AE Eindhoven (NL)**

(74) Representative: **Philips Intellectual Property & Standards
High Tech Campus 5
5656 AE Eindhoven (NL)**

(54) **TUMOR CLASSIFICATION**

(57)　A method and system for classifying tumor type from a pathology slide image. At least one pathology slide segment is provided to a plurality of CNN-based learning algorithms at a plurality of different magnifications. A prediction result for each image segment is obtained from each CNN-based learning algorithm, and an individual confidence score is obtained for each prediction result. The prediction results and individual confidence scores are processed to obtain a final tumor classification decision and final confidence score.

FIG. 1

**Description**

FIELD OF THE INVENTION

The invention relates to the field of tumor classification, and in particular to classifying tumor types from pathology slides.

BACKGROUND OF THE INVENTION

Pathology slides, containing stained samples of body tissue, may be examined under a microscope in order to identify whether the sample contains tumor cells and to diagnose tumor type. The variation in and number of features of different organs, anatomical sites and lesions means that classifying tumors from pathology slides is time-consuming work for physicians and requires a high level of experience in order to classify a tumor accurately, making tumor classification from pathology slides particularly valuable.

However, tumor classification is an area of medicine in which physicians may be reluctant to rely on automation. The consequences of a false negative in tumor classification, that is, a pathology slide containing tumor cells that is classified as a normal tissue sample, are particularly severe, and physicians may be concerned that replacing the expertise of experienced pathologists with an automated tumor classification system would pose a risk to patients.

There is therefore a need for a tumor classification assistance system that is integrated into the clinical workflow to provide physicians with information that will enable them to classify tumors more efficiently and with improved confidence.

SUMMARY OF THE INVENTION

The invention is defined by the claims.

According to examples in accordance with an aspect of the invention, there is provided a computer-implemented method of classifying tumor type from a pathology slide image.

The method comprises: providing at least one pathology slide image segment at each of a plurality of different magnifications to a plurality of learning algorithms based on convolutional neural networks (CNN); obtaining a prediction result from each CNN-based learning algorithm for each image segment; obtaining an individual confidence score for each prediction result; obtaining a final tumor classification decision based on the prediction results; and calculating a final confidence score for the final tumor classification decision based on the individual confidence scores.

This method provides tumor classification by using several independent CNN-based learning algorithms to make a decision about the tumor type in a pathology slide image or image part. The use of several CNN-based learning algorithms reduces the risk of missed diagnosis, as the probability of all of the CNN-based learning algorithms making an incorrect decision is lower than the probability of any one CNN-based learning algorithm making an incorrect decision.

This method also recognizes that different levels of detail can be observed at different magnifications and that results at different magnifications can be compared in order to improve the accuracy of the tumor classification. The at least one pathology slide image segment is therefore provided to the plurality of CNN-based learning algorithms at a plurality of different magnifications, and the tumor classification decisions at different magnifications may be used to reaffirm the final tumor classification decision.

In addition to providing a tumor classification decision, the method determines a confidence score for the decision. This confidence score provides additional information to the physician, which may give an indication of whether the clinician needs to examine the pathology slide manually in order to finalize the tumor classification. The efficiency of the tumor classification process is therefore increased, by reducing the time spent by physicians examining pathology slides that could be classified automatically with a high level of confidence while ensuring that decisions with low confidence scores are brought to the attention of physicians.

In other words, at least one pathology slide image segment is provided to a plurality of CNN-based learning algorithms to obtain a plurality of prediction results, thus improving the accuracy of the tumor classification decision obtained from the plurality of the prediction results. An individual confidence score is obtained for each prediction result; the individual confidence scores are combined to produce a final confidence score that may be supplied to a physician alongside the final tumor classification decision, thus ensuring that the physician is provided with sufficient information to decide whether he/she is able to trust the final tumor classification decision. The accuracy of the final tumor classification decision is further improved by providing the at least one pathology slide image to the plurality of CNN-based learning algorithms at a plurality of different magnifications; the final tumor classification decision may then be made by combining the tumor classification decisions at different magnifications.

In some embodiments, the number of image segments at each magnification depends on the magnification. In this way, more image segments can be used at higher magnifications such that each image segment has the same size.

Each CNN-based learning algorithm may be trained using a training algorithm configured to receive an array of training inputs and known outputs, wherein the training inputs may comprise pathology slide images or image segments and the known outputs may comprise tumor classifications. In this way, the CNN-based learning algorithms may be trained to output a tumor classification when provided with a pathology slide image or image segment.

In some embodiments, at least one CNN-based learning algorithm comprises a binary classifier trained to make a decision about the presence of a single tumor type, and wherein a plurality of CNN-based learning algorithms is used to make a decision for each of a plurality of tumor types. Dividing the classification into separate binary decisions simplifies the probability analysis as well as reducing noise in classification.

In some embodiments, the step of obtaining an individual confidence score for each prediction result comprises employing a softmax function. In this way, the individual confidence score for a prediction result corresponds to the probability that the prediction result is correct.

In some embodiments, the step of obtaining a final tumor classification decision comprises: determining a tumor classification decision for each image segment based on the prediction results for the image segment; determining an ensemble confidence score for each tumor classification decision based on the individual confidence scores for the prediction results, and the specificities and sensitivities of the CNN-based learning algorithms used to obtain the prediction results; and processing the tumor classification decisions and ensemble confidence scores to obtain a final tumor classification.

In this way, a single tumor classification decision and a corresponding ensemble confidence score may be obtained for each image segment, where each tumor classification decision is based on the prediction results of a plurality of CNN-based learning algorithms to improve the accuracy of the tumor classification decision for each image segment. The ensemble confidence score takes into account the individual confidence scores of the prediction results and the specificity and sensitivity of the CNN-based learning algorithms, so that tumor classification decisions based on prediction results with lower individual confidence scores and/or from less accurate CNN-based learning algorithms have a lower ensemble confidence score that tumor classification decisions based on prediction results with higher individual confidence scores and/or from more accurate CNN-based learning algorithms.

The step of processing the tumor classification decisions and ensemble confidence scores may comprise weighting the tumor classification decision for each image segment according to at least one of: the ensemble confidence score for the tumor classification decision; and the number of neighboring image segments with the same tumor classification decision.

This recognizes that an image segment that genuinely contains tumor cells is likely to have neighboring image segments that also contain tumor cells. Giving less weight to image segments that have a different final decision to neighboring image segments, and/or to image segments that have low confidence scores, reduces the effect of false positives, that is, normal image segments that are judged to contain a tumor.

In some embodiments, the ensemble confidence score for each tumor classification decision is also based on a level of agreement between prediction results for each image segment. In this way, a high ensemble confidence score is given to decisions where there is agreement between the prediction results of the separate CNN-based learning algorithms and a lower confidence score is given when the CNN-based learning algorithms disagree with one another.

The calculation of the final confidence score may be based on the ensemble confidence scores for the tumor classification decisions.

According to examples in accordance with an aspect of the invention, there is provided a computer program comprising code means for implementing the method of any preceding claim when said program is run on a processing system.

According to another aspect of the invention, there is provided a processing system adapted to: provide at least one pathology slide image segment at each of a plurality of different magnifications to a plurality of CNN-based learning algorithms; obtain a prediction result from each CNN-based learning algorithm for each image segment; obtain an individual confidence score for each prediction result; obtain a final tumor classification decision based on the prediction results; and calculate a final confidence score for the final tumor classification decision based on the individual confidence scores.

In some embodiments, each CNN-based learning algorithm is a binary classifier trained to make a decision about the presence of a single tumor type, and wherein a plurality of CNN-based learning algorithms is used to make a decision for each of a plurality of tumor types.

In some embodiments, the processing system is adapted to: determine a tumor classification decision for each image segment based on the prediction results for the image segment; determine an ensemble confidence score for each tumor classification decision based on the individual confidence scores for the prediction results, and the specificities and sensitivities of the CNN-based learning algorithms used to obtain the prediction results; and process the tumor classification decisions and ensemble confidence scores to obtain a final tumor classification.

The processing system may be adapted to process the tumor classification decisions and ensemble confidence scores by: weighting the tumor classification decision for each image segment according to at least one of: the ensemble confidence score for the tumor classification decision; and the number of neighboring image segment with the same tumor classification decision; and processing the final decisions and ensemble confidence scores based on the weightings for each final decision.

There is also proposed a tumor classification system, comprising the processing system described above and a user interface configured to receive, from the processing system, and display the final tumor classification decision and final confidence score.

These and other aspects of the invention will be apparent from and elucidated with reference to the embodiments described hereinafter.

BRIEF DESCRIPTION OF THE DRAWINGS

For a better understanding of the invention, and to show more clearly how it may be carried into effect, reference will now be made, by way of example only, to the accompanying drawings, in which:

FIG. 1 is a flow diagram of a method for obtaining a tumor classification decision and corresponding confidence score, according to an embodiment of the invention.
FIG. 2 is a flow diagram of a method for obtaining a tumor classification decision and corresponding confidence score, according to another embodiment of the invention.
FIG. 3 is an illustration of the possible outcomes when three CNN-based learning algorithms each produce a prediction result for an image segment.
FIG. 4 is a diagram of a tumor classification system, according to an embodiment of the invention.

DETAILED DESCRIPTION OF THE EMBODIMENTS

The invention will be described with reference to the Figures.

It should be understood that the detailed description and specific examples, while indicating exemplary embodiments of the systems and methods, are intended for the purposes of illustration only and are not intended to limit the scope of the invention. These and other features, aspects and advantages of the systems and methods of the present invention will become better understood from the following description, appended claims and accompanying drawings. It should be understood that the same reference numerals are used throughout the Figures to indicate the same or similar parts.

According to a concept of the invention, there is proposed a method and system for classifying tumor type from a pathology slide image. At least one pathology slide segment is provided to a plurality of CNN-based learning algorithms at a plurality of different magnifications. A prediction result for each image segment is obtained from each CNN-based learning algorithm, and an individual confidence score is obtained for each prediction result. The prediction results and individual confidence scores are processed to obtain a final tumor classification decision and final confidence score.

Embodiments are at least partly based on the realizations that the standard operation procedure for physicians of observing pathology slides at different magnifications can be employed by a tumor classification algorithm to increase the accuracy of tumor predictions, that obtaining tumor predictions from a plurality of tumor classification algorithms for a single pathology slide reduces the probability of missed diagnosis, and that providing physicians with a confidence score alongside an AI-derived tumor classification decision gives physicians more useful information than simply providing a computer result as a final decision, with no indication of the reliability of the decision.

Illustrative embodiments may, for example, be employed in digital pathology platforms as a clinical decision support system.

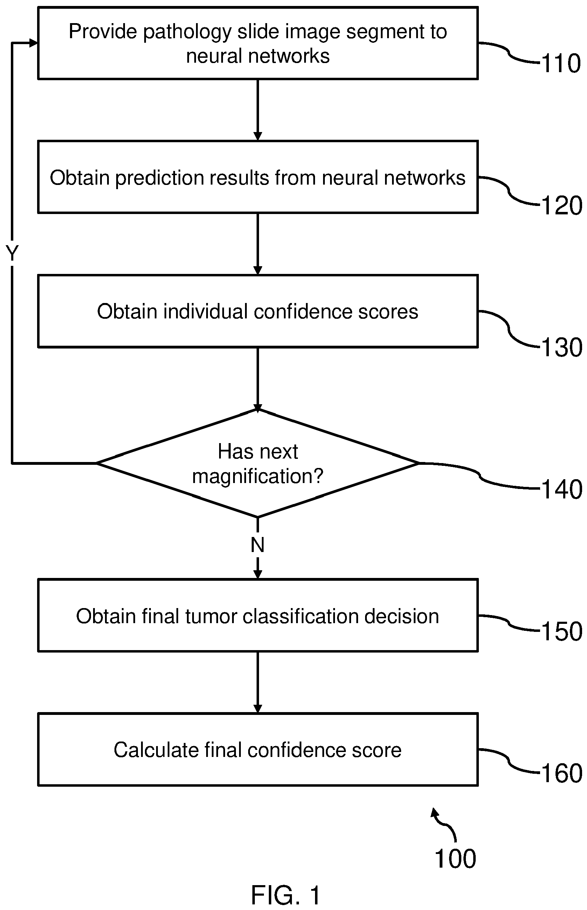

Figure 1 illustrates a computer-implemented method 100 for obtaining a tumor classification decision and corresponding confidence score, according to an embodiment of the invention.

The method 100 begins with step 110, in which at least one segment of a pathology slide image at a first magnification is provided to a plurality of CNN-based learning algorithms.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

There are several types of neural network, such as convolutional neural networks (CNNs) and recurrent neural networks (RNNs). Embodiments of the present invention employ CNN-based learning algorithms, as CNNs have proved to be particularly successful at analyzing images, and are able to classify images with a much lower error rate than other types of neural network.

CNNs typically contain several layers, including a convolutional layer, a pooling layer, a fully connected layer and a softmax layer. The convolutional layer consists of a set of learnable filters and extracts features from the input. The pooling layer is a form of non-linear down-sampling, reducing the data size by combining the outputs of a plurality of neurons in one layer into a single neuron in the next layer. The fully connected layer connects each neuron in one layer to all the neurons in the next layer. The softmax layer determines a probability distribution for each output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, weightings of the mathematical operation of each neuron may be modified until the error converges. Known methods of modifying a neural network include gradient descent, backpropagation algorithms and so on.

The training input data entries for the plurality of CNN-based learning algorithms used in method 100 correspond to example digital pathology or high quality pathology slide images. The training output data entries correspond to tumor classifications.

The slide images used to train the plurality of CNN-based learning algorithms may be labeled with region and tumor type. Slide images that do not contain any tumor cells may be labeled as normal, and slide images that do contain tumor cells may be annotated by sketching the contour of the tumor region and labeled with the tumor type.

Several pre-processing methods may be employed to improve the training sample. Slide images from the same laboratory and the same digital pathology system may be normalized in display features such as gamma correction. The tissue in pathology slides is stained; the stained region of a slide image may be segmented using Otsu's method. This region may then be modified into a standard size.

The plurality of CNN-based learning algorithms may be trained using pathology slide images at a plurality of different magnifications. Magnified slide images may be cropped to a size that takes computing efficiency into account. Slide images containing tumor cells may be cropped only in the tumor region.

The plurality of CNN-based learning algorithms may be produced by modifying existing CNN-based learning algorithms, such as VGG, Inception and ResNet.

At step 120, prediction results from the at least one image segment at a first magnification are obtained from the plurality of CNN-based learning algorithms.

At step 130, an individual confidence score is obtained for each prediction result. The individual confidence score for each prediction result may be obtained by employing a softmax function.

At step 140, the computer determines whether there are further magnifications of the at least one image segment for which prediction results are required, and steps 110 to 140 are repeated for at least a second magnification of the at least one image segment.

Once prediction results have been obtained for all the required magnifications of the at least one image segment, step 150 is carried out, at which a final tumor classification decision is obtained based on the prediction results.

At step 160, a final confidence score for the final tumor classification decision is calculated based on the individual confidence scores.

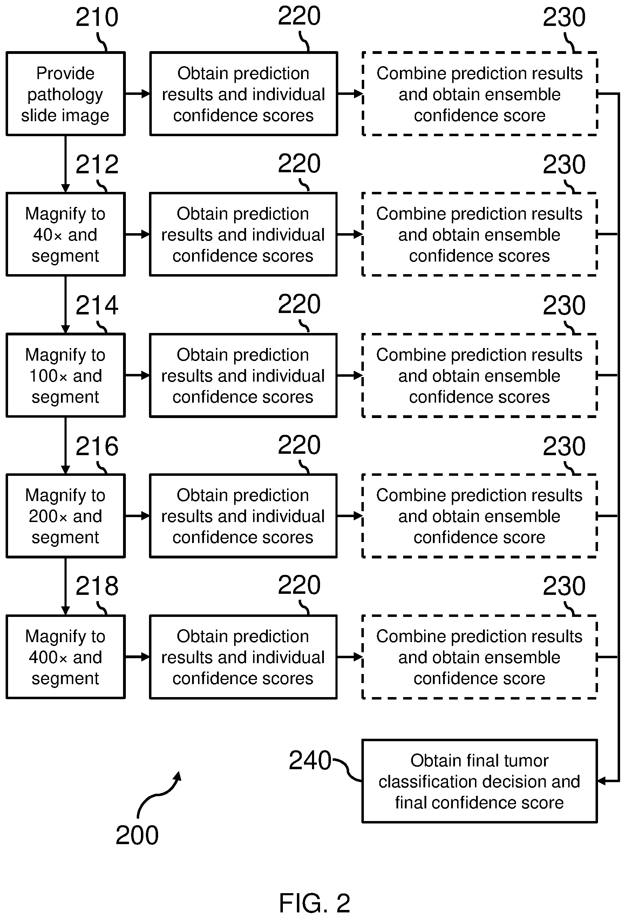

Figure 2 illustrates another computer-implemented method 200 for obtaining a tumor classification decision and corresponding confidence score, according to an embodiment of the invention.

The method 200 begins with step 210, in which a pathology slide image is provided.

At steps 212 to 218, the image is magnified to a plurality of different magnifications and divided into a number of image segments at each magnification. In Figure 2, five different magnifications are used (original size, 40×, 100×, 200× and 400×), but other numbers of magnifications and values of magnification may be used.

The number of image segments that the pathology slide image is divided into may depend on the magnification. For example, the whole slide image may comprise one segment at the original image size, and at magnification 100× the image may be divided into 10,000 segments such that each segment has the same size as the original image.

For each magnification, step 220 is carried out, in which each image segment is provided to a plurality of CNN-based learning algorithms to obtain a prediction result and corresponding individual score from each CNN-based learning algorithm.

The method 200 may comprise a step 230, in which the prediction results for each image segment at each magnification are combined into a single tumor classification decision and an ensemble confidence score based on the individual confidence scores is determined for each tumor classification system. Examples of methods for determining tumor classification decisions and ensemble confidence scores are later provided.

At step 240, the final tumor classification decision and final confidence score are obtained. The step of obtaining the final tumor classification decision and final confidence score may comprise processing the tumor classification decisions and ensemble confidence scores at each magnification to determine a result and confidence score for the whole image at each magnification and combining the results and confidence scores for all magnifications to obtain a final tumor classification decision and final confidence score.

Other methods may be used to obtain the final tumor classification decision. For example, in embodiments where images at higher magnifications are divided into more segments, the tumor classification decisions and ensemble confidence scores for segments at the highest magnification that comprise a single segment at the second highest magnification may be processed to obtain a single result and confidence score. This result and confidence score may be combined with the tumor classification decision and ensemble confidence score for the segment at the second highest magnification. This method may be repeated until the lowest magnification is reached.

Processing the tumor classification decisions and ensemble confidence scores may comprise weighting the tumor classification decision for each image segment according to at least one of the ensemble confidence score for the tumor classification decision and the number of neighboring image segments with the same tumor classification decision. Image segments that have high ensemble confidence scores may be given more weight than image segments with lower ensemble confidence scores. Image segments with a high number of neighboring image segments that have the same tumor classification decision may be given more weight than image segments with a lower number of neighboring image segments that have the same tumor classification decision. Examples of methods for weighting tumor classification decisions are later provided.

Returning to step 230, the tumor classification decision for each image segment may be obtained by determining which decision has a majority among the prediction results from the plurality of CNN-based learning algorithms. The ensemble confidence score for each tumor classification decision may be determined from the individual confidence scores for the prediction results, and the specificities and sensitivities of the CNN-based learning algorithms used to obtain the prediction results. The specificity and sensitivity of each CNN-based learning algorithm may be determined by testing the CNN-based learning algorithm.

In some embodiments, each CNN-based learning algorithm comprises a binary classifier trained to make a decision about the presence of a single tumor type, and a plurality of CNN-based learning algorithms are used to make a decision for each of a plurality of tumor types. This simplifies the probability analysis and reduces noise in classification.

The output of each CNN-based learning algorithm is therefore either positive or negative for a particular tumor type. The tumor classification decision for each image segment for each tumor type may be determined from whether the majority of prediction results for the image segment for that tumor type are positive or negative. For example, when three CNN-based learning algorithms are used for each tumor type, the tumor classification decision for an image segment may be positive when all three CNN-based learning algorithms or two out of the three CNN-based learning algorithms return a positive result and negative when all three CNN-based learning algorithms or two out of the three CNN-based learning algorithms return a negative result.

The calculation used to determine the ensemble confidence score for each image segment may depend on the prediction results for the image segment and the level of agreement between them.

For example, when three CNN-based learning algorithms are used for each tumor type, there are four possible outcomes for each image segment: all three prediction results are positive, two prediction results are positive and one is negative, two prediction results are negative and one is positive, and all three prediction results are negative. A different calculation may be used to determine the ensemble confidence score for each of these outcomes, such that image segments have a higher ensemble confidence score when there is agreement between the prediction results.

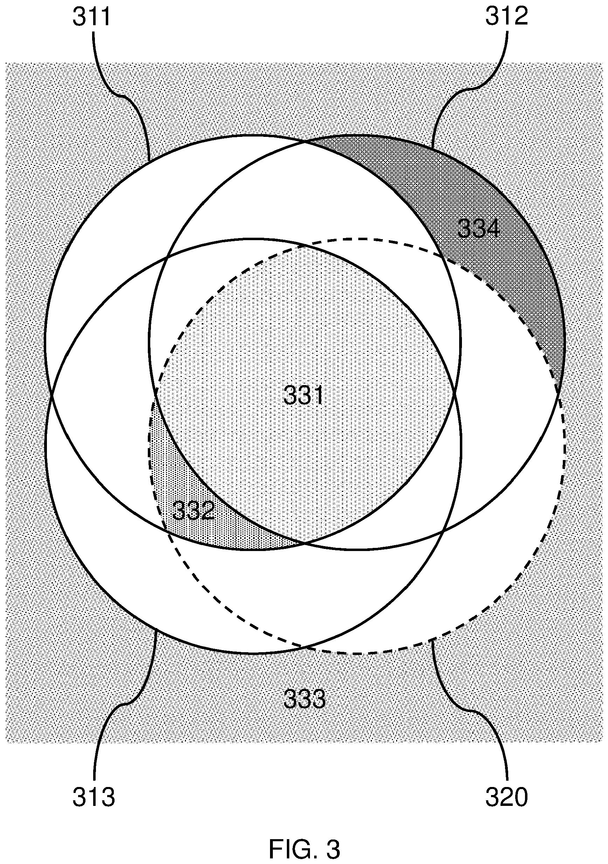

Figure 3 illustrates the possible outcomes when three CNN-based learning algorithms each produce a prediction result for an image segment. The circles 311, 312 and 313 each encompass positive prediction results from a different CNN-based learning algorithm. The circle 320 encompasses genuine positive cases.

The first possible outcome is that all three prediction results are positive for an image segment, resulting in a positive tumor classification decision for the image segment. The ensemble confidence score may then be calculated to give an indication of the probability that the three positive prediction results are correct. The area 331 is the area in which all four circles overlap. This represents genuine positive cases where a positive prediction result is obtained from all three CNN-based learning algorithms. The ensemble confidence score when all three prediction results are positive may be calculated by finding the probability that the case falls within area 331.

The probability that all three results are false positive is $\prod^{i=a,b,c}(1 - Sp(i))$, where $a$, $b$ and $c$ are the three results and $Sp(i)$ is the specificity of each result. The probability that this is not the case, and therefore the probability that the image segment is a true positive is therefore $[1 - \prod^{i=a,b,c}(1 - Sp(i))]$. The ensemble confidence score, $EnScore$, may be calculated by multiplying this probability by the mean of multiplying the sensitivity $Se(i)$ and individual confidence score $Co(i)$ of each result, $[\sum^{i=a,b,c} Se(i) \cdot Co(i)]/n$, where $n$ is the number of CNN-based learning algorithms used:

$$EnScore = [1 - \prod^{i=a,b,c}(1 - Sp(i))] \cdot \left(\sum^{i=a,b,c} Se(i) \cdot Co(i)\right)/n \qquad [1]$$

The second possible outcome is that two of the prediction results are positive for an image segment while the third prediction result is negative. This outcome again results in a positive tumor classification decision for the image segment. The ensemble confidence score in this case represents the probability that the positive prediction results are correct and the negative prediction result is incorrect, that is, the probability that the case falls within area 332, which represents genuine positive cases where a positive prediction result is obtained from two of the CNN-based learning algorithms, but not the third.

The ensemble confidence score in this case may be calculated by modifying Formula 1 such that sensitivity is replaced with specificity and vice versa for the negative prediction result:

$$EnScore = [1 - \prod^{i=a,b}(1 - Sp(i))] \cdot [1 - Se(c)] \cdot [\sum^{i=a,b} Se(i) \cdot Co(i) - Sp(c) \cdot Co(c)] / n \qquad [2]$$

The third possible outcome is that all three prediction results for an image segment are negative, resulting in a negative tumor classification for the image segment. The ensemble confidence score in this case represents the probability that the three negative prediction results are correct, that is, the probability that the case falls within area 333, which represents genuine negative cases where a negative result is obtained from each CNN-based learning algorithm.

The ensemble confidence score in this case may be calculated by modifying Formula 1 such that sensitivity is replaced with specificity and vice versa for all three results:

$$EnScore = (1 - \prod^{i=a,b,c}(1 - Se(i)) \cdot [\sum^{i=a,b,c} Sp(i) \cdot Co(i)] / n \qquad [3]$$

The fourth outcome is that two of the prediction results for an image segment are negative and the third is positive. This outcome results in a negative tumor classification decision for the image segment. The ensemble confidence score in this case represents the probability that the two negative prediction results are correct and the positive prediction result is incorrect, that is, the probability that the case falls within area 334, which represents genuine negative cases where a negative prediction result is obtained from two of the CNN-based learning algorithms, but not the third.

The ensemble confidence score in this case may be calculated by modifying Formula 1 such that sensitivity is replaced with specificity and vice versa for the negative prediction results:

$$EnScore = [1 - \prod^{i=a,b}(1 - Se(i))] \cdot [1 - Sp(c)] \cdot [\sum^{i=a,b} Sp(i) \cdot Co(i) - Se(c) \cdot Co(c)] / n \qquad [4]$$

In some embodiments, the calculated ensemble confidence score for an image segment is used to weight the tumor classification decision for the image segment. This may be done by employing the transformation $e^{(\pm)EnScore}$, where positive tumor classifications take the positive value of the ensemble confidence score and negative tumor classifications take the negative value. In this way, the exponential function results in values over 1 for positive tumor classification decisions and values between 0 and 1 for negative tumor classification decisions, thus lifting the weight of positive image segments to better detect positive slide images.

The tumor classification decision may also be weighted according to the number of neighboring image segments with the same tumor classification decision. For an image segment that has neighboring segments on all sides, the image segment has 8 neighboring segments. A coefficient weight $w_m$ ($m \in [0,8]$) may be assigned, where $m$ is the number of neighboring segments with the same tumor classification decision. The coefficient weights $w_m$ ($m \in [0,8]$) may be determined from a logistic regression model using training data.

In some embodiments, both the ensemble confidence score and the coefficient weight based on the number of neighboring segments with the same tumor classification decision may be used to weight the image segments:

$$LiScore = \sum^{i=0,1,2\ldots} w_m \cdot e^{(\pm)EnScore(i)}, m \in [0,8] \qquad [5]$$

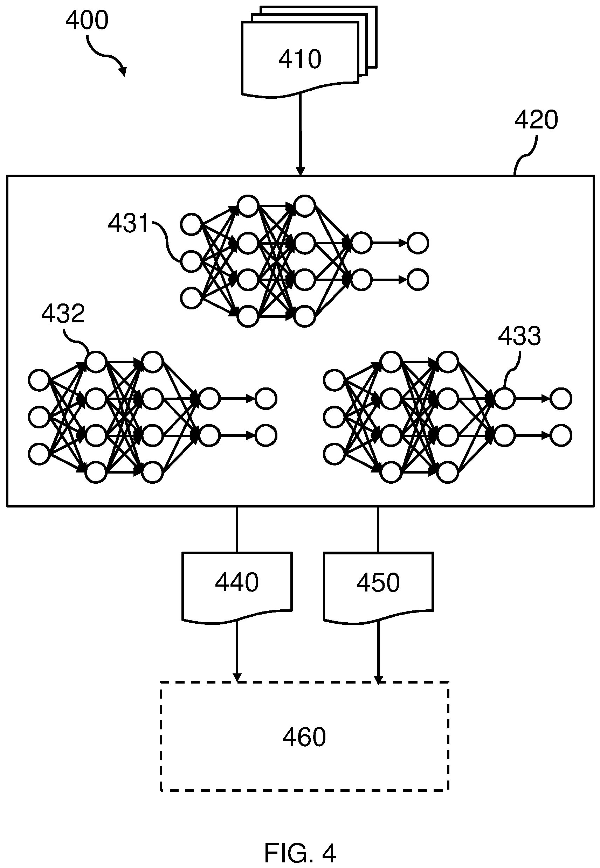

Figure 4 illustrates a tumor classification system 400, comprising a processing system 420, according to an embodiment of the invention. The processing system 420 is, itself, an embodiment of the invention.

At least one pathology slide image segment at each of a plurality of different magnifications 410 is provided to the processing system, which provides each image segment 410 to a plurality of CNN-based learning algorithms 431, 432 and 433. In Figure 4, three CNN-based learning algorithms are used to classify each image segment 410, but any number of CNN-based learning algorithms may be used. One or more of the CNN-based learning algorithms 431, 432 and 433 may be a binary classifier trained to make a decision about the presence of a single tumor type, and a plurality of CNN-based learning algorithms may be used to make a decision for each of a plurality of tumor types.

The processing system 420 obtains a prediction result from each CNN-based learning algorithm 431, 432 and 433 for each image segment, and an individual confidence score for each prediction result.

The processing system 420 may obtain a final tumor classification decision 440 based on the prediction results and a final confidence score 450 based on the individual confidence scores using any of the described methods.

The tumor classification system 400 may further comprise a user interface 460, which may be configured to receive the final tumor classification 440 and final confidence score 450 from the processing system 420, and to display the final tumor classification decision 440 and final confidence score 450. This may be a single final tumor classification decision and final confidence score, comprising a tumor type diagnosis or a decision that the pathology slide does not contain a tumor, or in examples where the CNN-based learning algorithms 431, 432 and 433 are binary classifiers, the user interface 460 may display a table comprising the final tumor classification decision 440 and final confidence score 450 for each tumor type.

In some embodiments, the user interface 460 may display additional information, such as the locations within the pathology slide image that have been judged to contain tumor cells.

In some embodiments, the processing system 420 is adapted to determine a tumor classification decision for each image segment 410 based on the prediction results for the image segment 410; determine an ensemble confidence score for each tumor classification decision based on the individual confidence scores for the prediction results, and the specificities and sensitivities of the CNN-based learning algorithms 431, 432 and 433 used to obtain the prediction results using any of the described methods; and process the tumor classification decisions and ensemble confidence scores to obtain a final tumor classification 440.

In some embodiments, the processing system 420 is further adapted to process the tumor classification decisions and ensemble confidence scores by weighting the tumor classification decision for each image segment 410 according to at least one of: the ensemble confidence score for the tumor classification decision; and the number of neighboring image segments with the same tumor classification decision; and by processing the tumor classification decisions and ensemble confidence scores based on the weightings for each tumor classification decision.

It will be understood that the disclosed methods are computer-implemented methods. As such, there is also proposed a concept of a computer program comprising code means for implementing any described method when said program is run on a processing system.

The skilled person would be readily capable of developing a processor for carrying out any herein described method. Thus, each step of a flow chart may represent a different action performed by a processor, and may be performed by a respective module of the processing processor.

As discussed above, the system makes use of a processor to perform the data processing. The processor can be implemented in numerous ways, with software and/or hardware, to perform the various functions required. The processor typically employs one or more microprocessors that may be programmed using software (e.g. microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted that the term "adapted to" is intended to be equivalent to the term "configured to". Any reference signs in the claims should not be construed as limiting the scope.

**Claims**

1. A computer-implemented method of classifying tumor type from a pathology slide image, the computer-implemented method comprising:

   providing at least one pathology slide image segment at each of a plurality of different magnifications (410) to a plurality of CNN-based learning algorithms (431, 432, 433);
   obtaining a prediction result from each CNN-based learning algorithm (431, 432, 433) for each image segment (410);
   obtaining an individual confidence score for each prediction result;
   obtaining a final tumor classification decision (440) based on the prediction results; and
   calculating a final confidence score (450) for the final tumor classification decision (440) based on the individual confidence scores.

2. The computer-implemented method of claim 1, wherein the number of image segments (410) at each magnification depends on the magnification.

3. The computer-implemented method of claim 1 or 2, wherein each CNN-based learning algorithm (431, 432, 433) is trained using a training algorithm configured to receive an array of training inputs and known outputs, wherein the training inputs comprise pathology slide images or image segments and the known outputs comprise tumor classifications.

4. The computer-implemented method of any of claims 1 to 3, wherein at least one CNN-based learning algorithm (431, 432, 433) comprises a binary classifier trained to make a decision about the presence of a single tumor type, and wherein a plurality of CNN-based learning algorithms is used to make a decision for each of a plurality of tumor types.

5. The computer-implemented method of any claims 1 to 4, wherein the step of obtaining an individual confidence score for each prediction result comprises employing a softmax function.

6. The computer-implemented method of any of claims 1 to 5, wherein the step of obtaining a final tumor classification decision (440) comprises:

   determining a tumor classification decision for each image segment (410) based on the prediction results for the image segment (410);
   determining an ensemble confidence score for each tumor classification decision based on the individual confidence scores for the prediction results, and the specificities and sensitivities of the CNN-based learning algorithms (431, 432, 433) used to obtain the prediction results; and
   processing the tumor classification decisions and ensemble confidence scores to obtain a final tumor classification (440).

7. The computer-implemented method of claim 6, wherein the step of processing the tumor classification decisions and ensemble confidence scores comprises weighting the tumor classification decision for each image segment (410) according to at least one of:

   the ensemble confidence score for the tumor classification decision; and
   the number of neighboring image segments with the same tumor classification decision.

8. The computer-implemented method of claim 6 or 7, wherein the ensemble confidence score for each tumor classification decision is also based on a level of agreement between prediction results for each image segment (410).

9. The computer-implemented method of any of claims 6 to 8, wherein the calculation of the final confidence score (450) is based on the ensemble confidence scores for the tumor classification decisions.

10. A computer program comprising code means for implementing the method of any preceding claim when said program is run on a processing system.

11. A processing system (420) adapted to:

provide at least one pathology slide image segment at each of a plurality of different magnifications (410) to a plurality of CNN-based learning algorithms (431, 432, 433);
obtain a prediction result from each CNN-based learning algorithm (431, 432, 433) for each image segment (410);
obtain an individual confidence score for each prediction result;
obtain a final tumor classification decision (440) based on the prediction results; and
calculate a final confidence score (450) for the final tumor classification decision (440) based on the individual confidence scores.

12. The processing system (420) of claim 11, wherein at least one CNN-based learning algorithm (431, 432, 433) comprises a binary classifier trained to make a decision about the presence of a single tumor type, and wherein a plurality of CNN-based learning algorithms is used to make a decision for each of a plurality of tumor types.

13. The processing system (420) of claim 11 or 12, further adapted to:

determine a tumor classification decision for each image segment (410) based on the prediction results for the image segment (410);
determine an ensemble confidence score for each tumor classification decision based on the individual confidence scores for the prediction results, and the specificities and sensitivities of the CNN-based learning algorithms (431, 432, 433) used to obtain the prediction results; and
process the tumor classification decisions and ensemble confidence scores to obtain a final tumor classification decision (440).

14. The processing system (420) of claim 13, wherein the processing system (420) is adapted to process the tumor classification decisions and ensemble confidence scores by:

weighting the tumor classification decision for each image segment (410) according to at least one of:

the ensemble confidence score for the tumor classification decision; and
the number of neighboring image segments with the same tumor classification decision; and

processing the tumor classification decisions and ensemble confidence scores based on the weightings for each tumor classification decision.

15. A tumor classification system (400), comprising:

the processing system (420) of any of claims 11 to 14; and
a user interface (460) configured to receive, from the processing system, and display the final tumor classification decision (440) and final confidence score (450).

Provide pathology slide image segment to neural networks —110

Obtain prediction results from neural networks —120

Obtain individual confidence scores —130

Has next magnification? —140

Y

N

Obtain final tumor classification decision —150

Calculate final confidence score —160

100

FIG. 1

FIG. 2

FIG. 3

FIG. 4

Europäisches
Patentamt

European
Patent Office

Office européen
des brevets

**EUROPEAN SEARCH REPORT**

Application Number

EP 20 15 3515

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2018/156133 A1 (GOOGLE LLC [US]) 30 August 2018 (2018-08-30) | 1,3-15 | INV. G16H50/20 G06T7/00 |
| Y | * page 2, line 6 - line 15 * <br> * page 9, line 25 - page 10, line 11 * <br> * page 14, line 13 - line 28 * | 2 | |
| X | US 2019/156159 A1 (KOPPARAPU KAVYA VENKATA KOTA SAI [US]) 23 May 2019 (2019-05-23) | 1,3-15 | |
| Y | * paragraphs [0005], [0011], [0068], [0096], [0115], [0135], [0137], [0158]; figures 1, 6 * | 2 | |
| X | US 2019/183429 A1 (SUNG KYUNG HYUN [US] ET AL) 20 June 2019 (2019-06-20) | 1,3-15 | |
| Y | * paragraphs [0005], [0016], [0044], [0045], [0053]; figure 2 * | 2 | |
| Y | US 2018/322327 A1 (SMITH RICHARD BOYD [US] ET AL) 8 November 2018 (2018-11-08) <br> * paragraph [0048]; figure 3 * | 2 | |
| A | WO 2018/152248 A1 (DIGNITY HEALTH [US]) 23 August 2018 (2018-08-23) <br> * paragraphs [0003], [0010], [0025] - [0027], [0048], [0086] * | 1-15 | TECHNICAL FIELDS SEARCHED (IPC) <br> G16H <br> G06T <br> G06K |
| X | SARA HOSSEINZADEH KASSANI ET AL: "Classification of Histopathological Biopsy Images Using Ensemble of Deep Learning Networks", ARXIV.ORG, CORNELL UNIVERSITY LIBRARY, 201 OLIN LIBRARY CORNELL UNIVERSITY ITHACA, NY 14853, 26 September 2019 (2019-09-26), XP081483444, * Abstract; Chapter 4.1 Datasets description * | 1-15 | |

The present search report has been drawn up for all claims

| Place of search | Date of completion of the search | Examiner |
|---|---|---|
| Munich | 19 June 2020 | Schmidt, Karsten |

## ANNEX TO THE EUROPEAN SEARCH REPORT
## ON EUROPEAN PATENT APPLICATION NO.

EP 20 15 3515

This annex lists the patent family members relating to the patent documents cited in the above-mentioned European search report.
The members are as contained in the European Patent Office EDP file on
The European Patent Office is in no way liable for these particulars which are merely given for the purpose of information.

19-06-2020

| Patent document cited in search report | | Publication date | Patent family member(s) | | Publication date |
|---|---|---|---|---|---|
| WO 2018156133 | A1 | 30-08-2018 | CN 110337644 A | | 15-10-2019 |
| | | | EP 3570753 A1 | | 27-11-2019 |
| | | | US 2020066407 A1 | | 27-02-2020 |
| | | | WO 2018156133 A1 | | 30-08-2018 |
| US 2019156159 | A1 | 23-05-2019 | NONE | | |
| US 2019183429 | A1 | 20-06-2019 | EP 3432784 A1 | | 30-01-2019 |
| | | | US 2019183429 A1 | | 20-06-2019 |
| | | | WO 2017165801 A1 | | 28-09-2017 |
| US 2018322327 | A1 | 08-11-2018 | NONE | | |
| WO 2018152248 | A1 | 23-08-2018 | CA 3053368 A1 | | 23-08-2018 |
| | | | US 2020129263 A1 | | 30-04-2020 |
| | | | WO 2018152248 A1 | | 23-08-2018 |

EPO FORM P0459

For more details about this annex : see Official Journal of the European Patent Office, No. 12/82